# EUROPEAN PATENT APPLICATION

(11) **EP 2 298 725 A1**
(43) Date of publication of application: **23.03.2011**
(21) Application number: 09762357.3
(22) Date of filing: 21.05.2009
(51) Int. Cl.: C07C 51/41, C07C 55/10, C07C 59/245, C07C 59/255, C07C 59/265, C07C 65/42, C07C 229/16

(54) **PROCESS FOR PRODUCING LIQUID METAL CHELATE COMPOUND AND METAL CHELATE COMPOUND**

(30) Priority: 09.06.2008 JP 2008150008
(71) Applicant: SUGIMOTO, Mikio, Yamaguchi 759-0207 (JP)
(72) Inventor: SUGIMOTO, Mikio, Yamaguchi 759-0207 (JP)
(74) Representative: Jones, Helen M.M.
(86) International application number: PCT/JP2009/059364
(87) International publication number: WO 2009/150927

(57) **Abstract**

The present invention is intended to provide a method for producing a liquid metal chelate compound which may be used as it is or diluted in water or the like, in a very simple procedure, in a short time, in a large quantity, and at a low cost, and to provide a metal chelate compound.

A metal is ground in a liquid containing an organic acid to form an unoxidized fine metal powder. For example, a metal is ground in a liquid containing the organic acid, or the organic acid is added after the metal is ground in a liquid, or the organic acid is added while grinding the metal in a liquid. The unoxidized fine metal powder is oxidized in the liquid containing an organic acid to generate a metal ion. As a result of this, the unoxidized fine metal powder is chemically bonded to the organic acid in the liquid thereby producing a liquid metal chelate compound. The liquid metal chelate compound is adsorbed by an absorber.

## Description

### BACKGROUND OF THE INVENTION

### Field of the Invention

The present invention relates to a method for producing a liquid metal chelate compound and a metal chelate compound.

### Description of the Related Art

Conventionally, a copper chelate compound of 8-oxyquinoline and anthranilic acid or substituted anthranilic acid is produced by reaction between anthranilic acid or substituted anthranilic acid, 8-oxyquinoline, and an inorganic copper salt in an aqueous alkaline solution in the absence of ethanol (for example, see Japanese Patent Application Laid-Open No. 8-176160).

The conventional method for producing a copper chelate compound includes, for example, adding anthranilic acid or substituted anthranilic acid, 8-oxyquinoline, and an inorganic copper salt in an aqueous alkaline solution, heating the mixture under stirring, allowing it to cool, collecting the precipitated crystals by filtration, washing and dehydrating the crystals, and then drying the crystals in a drier to obtain yellow green crystals.

The yellow green crystals are dissolved in water, and used for various applications.

Therefore, the conventional production method is based on the reaction between various compounds, and thus disadvantageously requires considerable time and effort, and an extremely high cost.

An object of the present invention is to provide a method for producing a liquid metal chelate compound which may be used as it is or diluted in water or the like, in a very simple procedure, in a short time, in a large quantity, and at a low cost, and to provide a metal chelate compound having prolonged effect.

### SUMMARY OF THE INVENTION

In order to solve the above problems, according to a first aspect of the present invention, there is provided a method for producing a liquid metal chelate compound, including grinding a metal in a liquid containing an organic acid to form an unoxidized fine metal powder, the unoxidized fine metal powder being oxidized in the liquid containing the organic acid to generate a metal ion, and the metal ion being chemically bonded to the organic acid in the liquid to form a liquid metal chelate compound.

According to a second aspect of the present invention, there is provided the method of the first aspect for producing a liquid metal chelate compound, wherein the metal is ground in a liquid containing the organic acid, or the organic acid is added after the metal is ground in a liquid, or the organic acid is added while grinding the metal in a liquid, thereby forming an unoxidized fine metal powder in the liquid containing the organic acid.

According to a third aspect of the present invention, there is provided a metal chelate compound composed of a liquid metal chelate compound absorbed by an absorber, the liquid metal chelate compound being produced by grinding a metal in a liquid containing an organic acid to form an unoxidized fine metal powder, the unoxidized fine metal powder being oxidized in the liquid containing an organic acid to generate a metal ion, and the metal ion being chemically bonded to the organic acid in the liquid to form a liquid metal chelate compound.

According to a fourth aspect of the present invention, there is provided the metal chelate compound of the third aspect, which is produced by grinding the metal in a liquid containing the organic acid, or adding the organic acid after the metal is ground in a liquid, or adding the organic acid while grinding the metal in a liquid, thereby forming an unoxidized fine metal powder in the liquid containing the organic acid.

According to the method of the first aspect for producing a liquid metal chelate compound, as described above, a metal is ground in a liquid containing an organic acid to form an unoxidized fine metal powder, the unoxidized fine metal powder being oxidized in the liquid containing an organic acid to generate a metal ion, and the metal ion being chemically bonded to the organic acid in the liquid to form a liquid metal chelate compound. Under the method, the unoxidized fine metal powder is oxidized in the organic acid solution to generate a metal ion in a short time with efficiency, and the metal ion immediately forms a chemical bond with the organic acid to produce a liquid metal chelate compound. Accordingly, the method produces a liquid metal chelate compound which may be used as it is or diluted in water or the like, in a very simple procedure, in a short time, in a large quantity, and at a low cost.

According to the method of the second aspect for producing a liquid metal chelate compound, a metal is ground in a liquid containing an organic acid. Under the method, the unoxidized fine metal powder is oxidized in the organic acid solution to generate a metal ion in a short time with efficiency, and the metal ion immediately forms a chemical bond with the organic acid to produce a liquid metal chelate compound. Alternatively, the organic acid is added after the metal is ground in a liquid, thereby controlling the concentration of the liquid metal chelate compound. Alternatively, the organic acid is added while grinding the metal, thereby producing a liquid metal chelate compound which may be used diluted in water or the like in a very simple procedure, in a short time, in a large quantity, and at a low cost.

The metal chelate compound according to the third aspect is composed of, as described above, a liquid metal chelate compound absorbed by an absorber. Therefore, the metal chelate compound slowly exudes from the absorber over a long period of time into water or the ground, and thus achieves its effect for a long time.

The metal chelate compound according to the fourth aspect is produced by grinding a metal in a liquid containing an organic acid. Under the method, the unoxidized fine metal powder is oxidized in the organic acid solution to generate a metal ion in a short time with efficiency, and the metal ion immediately forms a chemical bond with the organic acid to produce a liquid metal chelate compound. Alternatively, the organic acid is added after the metal is ground in a liquid, thereby controlling the concentration of the liquid metal chelate compound. Alternatively, the organic acid is added while grinding the metal, thereby producing a liquid metal chelate compound which may be used diluted in water or the like in a very simple procedure, in a short time, in a large quantity, and at a low cost.

Examples of the present invention are described below.

### Example 1

The method of Example 1 for producing a liquid metal chelate compound is described below. The method of Example 1 for producing a liquid metal chelate compound corresponds to first and second aspects of the present invention.

In Example 1, a metal is ground in a liquid containing an organic acid to form an unoxidized fine metal powder, the unoxidized fine metal powder being oxidized in the liquid containing an organic acid to generate a metal ion. As a result of this, the metal ion forms a chemical bond with the organic acid in the liquid to produce a liquid metal chelate compound. The formation of an unoxidized fine metal powder in a liquid containing an organic acid by grinding a metal is carried out by grinding a metal in a liquid containing an organic acid, or adding an organic acid after a metal is ground, or adding an organic acid after a metal is ground.

Examples of the organic acid such as carboxylic acid and hydroxy acid include fulvic acid, succinic acid, malic acid, tartaric acid, citric acid, ethylenediamine-tetraacetic acid (EDTA), and any organic acids to work as chelating agents. Various chelating agents are commonly used, such as carboxylic acid-based having a carboxyl group (-COOH), and hydroxy acid-based ones. Among them, most common organic acids are fulvic acid, suberic acid, malic acid, tartaric acid, citric acid, and ethylenediamine-tetraacetic acid (EDTA). They are versatile and easy to use, and thus are useful for the formation of metal chelate compounds used as microelement suppliers or polymerization catalysts.

It is well known that fulvic acid is derived from a natural humic substance. Fulvic acid is bound to iron in the soil to form an iron chelate compound referred to as iron fulvate which is supplied to natural animals and plants as an essential mineral. Succinic acid, malic acid, and tartaric acid are organic acids used as food additives or ingredients. Citric acid is a low-cost organic acid used as a food additive or ingredient highly beneficial for living bodies, and is contained in large quantities in Moromi (a Japanese fermented food), citrus fruits, and Umeboshi (Japanese pickled plums). Citric acid readily forms iron citrate. Ethylenediamine-tetraacetic acid (EDTA) is a versatile chelating agent which is commonly used. EDTA is readily available, and forms a chelate compound without an industrial process.

Examples of the metal material to be ground include magnesium (Mg), copper (Cu), iron (Fe), zinc (Zn), and silver (Ag). Other examples include alloys composed mainly of these metals, and a firmly bonded composite of any of these metals and carbon (C), for example, mixed sintered metal such as a metallic carbon brush, and a hybrid metal composite of carbon (C) and metal produced by a discharge plasma system (SPS sintering). Magnesium (Mg), copper (Cu), iron (Fe), and zinc (Zn) are trace minerals essential to living bodies, animals, and plants. They are generally sold as dietary supplements, and readily available at low costs.

Silver (Ag) is widely used in living spaces as an antiseptic or a sterilizer. In addition, silver colloid liquids have good electrical conductivity, and thus are used for passing a current. When a single metal or its alloy is turned into a fine powder, the surface area markedly increases. At the same time, the surface of the unoxidized fine powder instantaneously causes oxidation, and releases a metal ion into the liquid (organic chelating agent aqueous solution). A firmly bonded composite of carbon (C) and metal materials is preferable, because it supplies an unoxidized virgin fine powder of the firmly bonded carbon (C) and metal materials into the liquid (organic chelating agent aqueous solution) when the composite is turned into a fine powder in the liquid, so that the metal permanently releases a metal ion into the chelating agent aqueous solution based on the difference in the electronegativity and/or electrode potential of the carbon (C) and metal materials. When the firmly bonded composite of a metal and a carbon is used, any combinations other than the above example may be used.

For example, when a metal material composed mainly of iron (Fe) is ground to form a fine metal powder in an aqueous solution containing citric acid, which is a carboxylic acid used as an organic acid, the iron component is oxidized to generate an iron ion. The iron ion forms a chemical bond with citric acid to produce an iron citrate complex (liquid metal chelate compound).

The effect of Example 1 is described below.

In the method of Example 1 for producing the liquid metal chelate compound, as described above, a metal is ground in a liquid containing an organic acid to form an unoxidized fine metal powder. The unoxidized fine metal powder is oxidized in the liquid containing an organic acid to generate a metal ion, and the metal ion forms a chemical bond with the organic acid in the liquid to produce a liquid metal chelate compound. Under the method, the unoxidized fine metal powder is oxidized in the organic acid solution to generate a metal ion in a short time with efficiency, and the metal ion immediately forms a chemical bond with the organic acid to produce a liquid metal chelate compound.

Accordingly, a liquid metal chelate compound which may be used as it is or diluted in water or the like is produced in a very simple procedure, in a short time, in a large quantity, and at a low cost. The diluting water may be any water such as sea water or freshwater.

For example, when a metal is ground in a liquid containing an organic acid, an unoxidized fine metal powder is oxidized in the organic acid solution to generate a metal ion in a short time with efficiency, and the metal ion immediately forms a chemical bond with the organic acid to produce a liquid metal chelate compound. More specifically, a chelating agent aqueous solution containing an organic acid chelating agent is prepared so as to give an intended concentration in a water storage tank of any size or capacity, and a metal is ground in the liquid to form an unoxidized fine metal powder. The unoxidized fine metal powder is oxidized to generate a metal ion, and at the same time produces a metal chelate compound.
Alternatively, when an organic acid is added after a metal is ground in a liquid, the concentration of the liquid metal chelate compound is controlled by changing the amount of the metal ion to be generated. More specifically, a metal is ground in a liquid to form an unoxidized fine metal powder in a water storage tank of any size or capacity, and then an organic acid chelating agent is added to the tank, thereby producing a metal chelate compound solution having an intended concentration and containing a fine metal powder formed by grinding the metal for a desired period of time and in a desired amount.
Alternatively, when an organic acid is added while grinding a metal, a liquid metal chelate compound which may be used as it is or diluted in water or the like is produced in a very simple procedure, in a short time, in a large quantity, and at a low cost. More specifically, when an organic acid chelating agent is added while grinding a metal to form an unoxidized fine metal powder in a water tank or in a running liquid, the amount of the organic acid chelating agent is adjustable, and the metal may be ground while passing or discharging the liquid, and the fine metal powder thus formed is fed into the liquid to produce a metal chelate compound.

Another example is described below, wherein descriptions of components similar to Example 1 are omitted.

### Example 2

The metal chelate compound of Example 2 corresponds to the third and fourth aspects of the present invention.

The metal chelate compound of Example 2 is produced by grinding a metal in a liquid containing an organic acid to form an unoxidized fine metal powder. For example, a metal is ground in a liquid containing the organic acid, or the organic acid is added after the metal is ground in a liquid, or the organic acid is added while grinding the metal in a liquid, thereby forming an unoxidized fine metal powder from the ground metal in the liquid containing an organic acid. The unoxidized fine metal powder is oxidized in the liquid containing an organic acid to generate a metal ion, and the metal ion is chemically bonded to the organic acid to produce a liquid metal chelate compound. The liquid metal chelate compound is adsorbed by an absorber.

Examples of porous absorbers include plant carbon (charcoal, bamboo carbon, and other carbon), zeolite, diatomaceous earth, biodegradable foaming polymer, and pearlite.

Examples of absorbers having absorptivity and inclusiveness include fiber substances such as bagasse, bark, and straws, water-absorbing polymers, clay powders, and bentonite.

The effect of Example 2 is described below.

The metal chelate compound of Example 2 is composed of, as described above, a liquid metal chelate compound absorbed by an absorber. Therefore, the metal chelate compound slowly exudes from the absorber over a long period of time into water or the ground, and thus achieves its effect for a long time.

The absorber taken as an example is not a single inorganic substance but contains many minor components, and thus achieves synergistic effect together with the metal chelate compound.

The present invention will not limited to the above examples, and includes appropriate modifications without departing from the scope of the present invention.

For example, the liquid containing an organic acid may be a plant tar produced by plant carbonization, or its aqueous solution. In an alternative method, for example, an unoxidized fine powder is formed from a metal body in water such as sea water or freshwater, oxidized to form metal ion water, and then an organic acid to work as a chelating agent is added to produce a metallic chelate compound.

## Claims

1. A method for producing a liquid metal chelate compound, comprising grinding a metal in a liquid containing an organic acid to form an unoxidized fine metal powder, the unoxidized fine metal powder being oxidized in the liquid containing the organic acid to generate a metal ion, the metal ion being chemically bonded to the organic acid in the liquid to form a liquid metal chelate compound.

2. The method for producing a liquid metal chelate compound, according to claim 1, wherein the metal is ground in a liquid containing the organic acid, or the organic acid is added after the metal is ground in a liquid, or the organic acid is added while grinding the metal in a liquid, thereby forming an unoxidized fine metal powder in the liquid containing the organic acid.

3. A metal chelate compound composed of a liquid metal chelate compound absorbed by an absorber, the liquid metal chelate compound being produced by grinding a metal in a liquid containing an organic acid to form an unoxidized fine metal powder, the unoxidized fine metal powder being oxidized in the liquid containing an organic acid to generate a metal ion, and the metal ion being chemically bonded to the organic acid in the liquid to form a liquid metal chelate compound.

4. The metal chelate compound according to claim 3, which is produced by grinding the metal in a liquid containing the organic acid, or adding the organic acid after the metal is ground in a liquid, or adding the organic acid while grinding the metal in a liquid, thereby forming an unoxidized fine metal powder in the liquid containing the organic acid.
